(19) Europäisches Patentamt
European Patent Office
Office européen des brevets

(11) Publication number: 0 145 377 A2

# (12) EUROPEAN PATENT APPLICATION

(21) Application number: 84308090.4

(22) Date of filing: 21.11.84

(51) Int. Cl.4: C 07 C 93/14

(30) Priority: 21.11.83 IT 2378983

(43) Date of publication of application: 19.06.85 Bulletin 85/25

(84) Designated Contracting States: BE CH DE FR GB LI NL

(71) Applicant: Montedison S.p.A., 31, Foro Buonaparte, I-20121 Milan (IT)

(72) Inventor: Beretta, Maurizio Augusto, 75, via Valvassori Peroni, Milan (IT)
Inventor: Bausani, Giovanni, 4, via Tiziano, Trecate Novara (IT)
Inventor: Bottaccio, Giorgio, 16, via Manin, Novara (IT)
Inventor: Campolmi, Stefano, 27, via A. Costa, Novara (IT)
Inventor: Montanari, Fernando, 29, via L. Muratori, Milan (IT)

(74) Representative: Whalley, Kevin et al, Marks & Clerk 57/60 Lincoln's Inn Fields, London WC2A 3LS (GB)

## (54) Process for preparing 3,4'-Amino-Nitro-Diphenylether.

(57) A process for preparing 3,4′-amino-nitro-diphenylether in a heterogenous phase, according to the phase-transfer catalysis method, in which para-chloro-nitrobenzene is reacted with an alkali metal or alkaline-earth metal salt, preferably sodium or potassium, of meta-amino-phenol, preferably at a temperature ranging from 50 °C to 120 °C, in the presence of a phase-transfer catalyst, preferably (1) an onium salt, more preferably a quaternary alkylammonium salt $QR_4Y$ wherein Q is selected from N, P, As, Sb etc., R is an alkyl radical containing up to 20 carbon atoms and Y is Cl, Br, or I, or (2) a crown ether, in a two-phase system consisting of (a) an organic phase represented by the para-chloro-nitrobenzene, optionally in an organic solution, and (b) by the meta-amino-phenol in the form of an alkali metal or alkaline-earth metal salt in an aqueous solution which is substantially immiscible with the first phase.

In this way, 3,4′-amino-nitro-diphenylether is obtained, which is a particularly useful intermediate for preparing fibres.

EP 0 145 377 A2

"PROCESS FOR PREPARING 3,4' - AMINO-NITRO-DIPHENYLETHER"

The present invention relates to a process for preparing 3,4' -amino-nitro-diphenylether, in particular according to a catalytic method in a heterogeneous phase.

More particularly, the present invention relates to a process for preparing 3,4' -amino-nitro-diphenylether by means of a method based on so-called phase-transfer catalysis, preferably by using quaternary organic salts and/or crown ethers better defined hereinafter.

The resulting compound is an interesting intermediate product, particularly useful in the synthesis of fibres, especially aramidic fibres of the "Kelvar" type. "Kelvar" is a Registered Trade Mark.

The method of carrying out catalyzed reactions according to the phase-transfer technique has been broadly developed and is well known.

As far as is known, however, it seems that there is limited knowledge in the field of application of this technique to the aromatic series, and in particular to the synthesis of 3,4' -amino-nitro-diphenylether.

According to a known technique (British patent No. 1,216,109), 3,4' -amino-nitro-diphenylether can be obtained only by condensation between para-chloro-nitrobenzene and meta-amino-phenol in the

presence of basic agents, by using expensive and not easily recoverable solvents, such as dimethylsulphoxide. On the other hand, the relatively high cost of the starting meta-amino-phenol makes it advisable to acquire a technique leading to particularly high yields and selectivities of such compound.

Thus, it is an aim of the present invention to provide a method of preparing 3,4'-amino-nitro-diphenylether according to the phase-transfer catalysis technique, which would be applicable on an industrial scale.

The present invention provides a process for preparing 3,4' -amino-nitro-diphenylether by reacting p-chloro-nitrobenzene with an alkali metal or alkaline-earth metal salt of m-amino-phenol or a precursor thereof, characterized in that the reaction is conducted in a diphase system consisting of an organic phase comprising the p-chloro-nitrobenzene and an aqueous phase containing the alkali metal or alkaline-earth metal salt of m-amino-phenol, in the presence of a phase-transfer catalyst.

Thus there is provided a process for preparing 3,4' -amino-nitro-diphenylether, by reaction in a phase-transfer system, in which para-chloro-nitrobenzene is reacted with meta-amino-phenol in the form of an alkali metal or alkaline-earth metal salt, preferably sodium or

potassium, preferably at a temperature approximately ranging from 50° to 120°C, more preferably from 90° to 110°C, and preferably substantially at atmospheric pressure, characterized in that the reaction is conducted in the presence of a phase-transfer catalyst, and more particularly in the presence of an onium salt, preferably a quaternary alkylammonium salt of formula $QR_4Y$, where Q is an atom of N, P, As, Sb, etc., R is an alkyl radial containing up to 20 carbon atoms and Y is a halogen selected from chlorine, bromine and iodine, or a crown ether, in a diphase system consisting of (a) a first organic phase composed of para-chloro-nitrobenzene, optionally dissolved in an organic solvent selected from aromatic and/or aliphatic hydrocarbons and chloroderivatives thereof, the second phase (b) being composed of meta-amino-phenol in the form of an alkali metal or alkaline-earth metal salt as defined hereinabove, in an aqueous solution substantially immiscible with the first phase.

The reaction can be schematically expressed by the following equation:

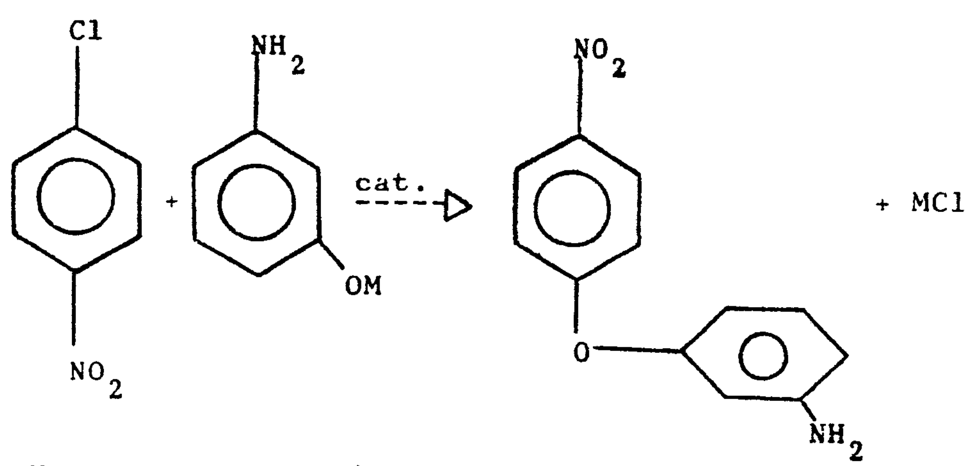


(M = Na, K, etc. .....).

The alkali metal or alkaline-earth metal salt of meta-amino-phenol can be substituted by a pre-cursor thereof: the meta-amino-phenol itself and the alkali metal oxide or hydrate in a substantially stoichiometric amount with respect to each other, and preferably in defect in respect of para-chloro-nitrobenzene, and precisely in a molar ratio in respect of para-chloro-nitrobenzene ranging from 1:1 to 1:10, approximately.

As mentioned hereinbefore, the para-chloro-nitrobenzene may be utilized either as such or in an organic solution. The solvent is selected from aromatic and/or aliphatic hydrocarbons and chloroderivatives thereof, such as toluene or chlorobenzene.

The reaction terminates in a time generally ranging from about 2 to about 50 hours, depending on the parameters employed, i.e. temperature, concentration, catalyst type.

The molar ratio between the para-chloro-nitrobenzene and the alkali metal or alkaline-earth metal salt of meta-amino-phenol is preferably substantially equimolar; however, an excess of para-chloro-nitrobenzene up to a molar ratio of 10:1 is permissible, and a molar ratio ranging from 2:1 to 4:1 is preferable.

The phase-transfer catalyst suitably comprised by the onium salt, preferably alkylammonium salt $NR_4Y$, or the

crown ether, is preferably added according to a molar ratio with respect to the starting meta-amino-phenol in a range of from 0.01 to 0.2, approximately.

The term onium salts means the so-called salts of quaternary ammonium, phosphonium, etc. having, as is known to those skilled in the art, a catalytic activity for the purpose of the phase-transfer reaction. Such salts can be comprised of the formula $QR_4Y$, in which Q represents an atom of nitrogen, phosphorus, arsenic or antimony respectively for the salts of ammonium, phosphonium, etc, and R and Y are as defined hereinabove.

For practical reasons, the preferred salts are the quaternary ammonium salts previously defined.

Particularly effective results may be obtained, according to the present invention, when using as onium quaternary salts those in which the alkyl radical contains at least 4 carbon atoms, in particular tetrabutylammonium bromide, tricaprylmethylammonium chloride, trilaurylmethylammonium chloride, and tetrabutyl-phosphonium chloride and bromide.

The crown ethers employed are cyclic polyethers which are known as complexing agents for alkali metals. Effective crown ethers have proved to be, for example, dicyclohexyl-18-crown-6 and dibenzo-18-crown-6.

It is of course also possible to use, as phase-transfer catalysts, other compounds, namely those which are usually utilized in said technique.

The reaction is suitably conducted in a non-oxidizing atmosphere, which can be obtained according to conventional methods with nitrogen, argon, etc.

The process according to a preferred embodiment of the invention is carried out as follows.

Into a reactor, under a nitrogen atmosphere, there are introduced the para-chloro-nitrobenzene, the organic solvent, if any, the quaternary onium salt or the crown ether, and the meta-amino-phenol, whereafter the temperature is brought to the desired value.

Under stirring there is added an alkali metal or alkaline-earth metal hydrate or oxide in an aqueous solution, in a proper amount and time, while keeping the temperature at the predetermined value. The reaction is continued for the necessary time until completion.

The resulting 3,4' -amino-nitro-diphenylether is then separated according to conventional techniques, such as acid-basic extraction, crystallization, etc.

Due to the mild operative conditions and to the high selectivity of the desired product, the process is particularly advantageous.

Further advantages consist in the easy availability and low costs of the raw materials and in the simplicity of the equipment employed, since the process is practically carried out in one step only.

The process of the invention may be operated in

reactivity conditions which are equal to or even superior to those obtainable with the conventional processes, in which the same reaction is conducted in dipolar aprotic solvents such as dimethylsulphoxide.

In particular, the process exhibits the following advantages as compared with the known art:

1) it is possible to obtain a high catalyticy on the onium salt or crown ether;

2) it is possible to use aqueous solutions of the alkali metal or alkaline-earth metal salt of meta-amino-phenol;

3) it is possible not to use organic solvents, the dissolving function being carried out by the starting para-chloro-nitrobenzene.

The invention will be further described with reference to the following illustrative Examples.

Example 1

10.9 g (0.1 moles) of meta-amino-phenol, 47.1 g (0.3 moles) of para-chloro-nitrobenzene, and 3.8 cc (0.01 moles) of Aliquat 336 (R) (tricaprylmethylammonium chloride) were introduced, under a nitrogen atmosphere, into a 250 cc flask equipped with a stirrer, a thermometer, a gas bubbler, a reagent charting system and a reflux cooler. The reaction mass was made molten by increasing the temperature to about 100°C and, under stirring, in 2 hours, 6.6 g (0.1 moles) of KOH at 85%, dissolved in 60 cc of water, were added dropwise.

While keeping the system under reflux, at an internal temperature of about 105°C, the reaction was continued for a further 11 hours. On conclusion of the reaction, the system was acidified with dilute sulphuric acid until an acid pH was obtained, and the unreacted para-chloro-nitrobenzene was distilled in a vapour stream; thus, 30.6 g of para-chloro-nitrobenzene were recovered. The acid solution was hot treated with 1 g of activated carbon and with 2 g of diatomaceous earth. After filtration, the solution was made alkaline with aqueous soda; 3,4' -amino-nitro-diphenylether precipitated, whereafter it was filtered, washed and dried. Thus 22.4 g of product, corresponding to a yield of 97.4% referred to the starting meta-amino-phenol, were obtained; the selectivity referred to the reacted para-chloro-nitrobenzene was 92.7%.

Example 2

By operating as in example 1 and using 1.9 cc (0.005 moles) of Aliquat (R) 336, with a total reaction time (including dropping) of 24 hours, there were obtained 22 g of 3,4' -amino-nitro-diphenylether, corresponding to a yield of 95.7% referred to the starting meta-amino-phenol.

Example 3

By operating as in example 1 and using as a catalyst 1.5 g (0.0047 moles) of tetrabutylammonium bromide, 22.4

g of 3,4' -amino-nitro-diphenylether were obtained, which corresponded to a yield of 97.4% referred to the meta-amino-phenol.

<u>Example 4</u>

By operating as in example 1 and feeding, together with the para-chloro-nitrobenzene, 5 cc of xylene, 22.3 g of 3,4' -amino-nitro-diphenylether were obtained, which corresponded to a yield of 97% referred to the meta-amino-phenol.

<u>Examples 5-12</u>

By operating according to the manner of example 4 and varying some of the parameters, the results indicated in the following Table were obtained.

T A B L E

| Example N° | Para-chloro-nitro benzene | Catalyst | Base | Water | Time | Tempera-ture | Molar yield on meta--amino-phenol |
|---|---|---|---|---|---|---|---|
| 5 | 47,1 g (0,3 moles) | Aliquat 336 2,5 cc (0,0066 moles) | KOH 6,6 g (0,1 moles) | 60 cc | 13 h in total | 105°C | 93 % |
| 6 | 31,4 g (0,2 moles) | Aliquat 336 3,8 cc (0,01 moles) | " | " | " | " | 84 % |
| 7 | " | " | NaOH 4,0 g (0,1 moles) | " | " | " | 82 % |
| 8 | " | Tetrabutylammonium bromide 3.2 g (0,01 moles) | KOH 6,6 g (0,1 moles) | " | " | " | 100 % |
| 9 | " | Aliquat 336 (3,8 cc (0,01 moles) | " | 80 cc | " | " | 75 % |
| 10 | 15,7 g (0,1 moles) | " | " | 20 cc | " | 100°C | 67 % |
| 11 | 31,4 g (0,2 moles) | " | " | 60 cc | 9 h in total | 105°C | 75 % |
| 12 | 47,1 g (0,3 moles) | Tetrabutylammonium bromide (1,3 g (0,0041 moles) | " | " | 24 h in total | " | 97,4 % |

CLAIMS:

1. A process for preparing 3,4' -amino-nitro-diphenylether by reacting p-chloro-nitrobenzene with an alkali metal or alkaline-earth metal salt of m-amino-phenol or a precursor therof, characterized in that the reaction is conducted in a diphase system consisting of an organic phase comprising the p-chloro-nitrobenzene and an aqueous phase containing the alkali metal or alkaline-earth metal salt of m-amino-phenol, in the presence of a phase-transfer catalyst.

2. A process as claimed in claim 1, characterized in that the phase-transfer catalyst is an onium salt of formula $QR_4Y$ where Q is an atom of N, P, As or Sb, R is an alkyl radical containing up to 20 carbon atoms, and Y is a halogen selected from chlorine, bromine and iodine.

3. A process as claimed in claim 2, characterized in that the onium salt is selected from tetrabutylammonium bromide, tricaprylmethylammonium chloride, trilaurylmethylammonium chloride, tetrabutylphosphonium chloride and tetrabutylphosphonium bromide.

4. A process as claimed in claim 1, characterized in that the phase-transfer catalyst is a crown ether compound.

5. A process as claimed in claim 4, characterized in

that the crown ether compound is selected from dicyclohexyl-18-crown-6 and dibenzo-18-crown-6.

6. A process as claimed in any of claims 1 to 5, characterized in that the reaction is conducted at substantially ambient pressure and in an inert gas atmosphere.

7. A process as claimed in any of claims 1 to 6, characterized in that the reaction is conducted at a temperature from 50°C to 120°C.

8. A process as claimed in claim 7, characterized in that the reaction is conducted at a temperature from 90°C to 110°C.

9. A process as claimed in any of claims 1 to 8, characterized in that p-chloro-nitrobenzene and the alkali metal or alkaline-earth metal salt of m-amino-phenol are reacted in a molar ratio from 1:1 to 10:1.

10. A process as claimed in claim 9, characterized in that p-chloro-nitrobenzene and the alkali metal or alkaline-earth metal salt of m-amino-phenol are reacted in a molar ratio from 2:1 to 4:1.

11. A process as claimed in any of claims 1 to 10, characterized in that the phase-transfer catalyst is added in a molar ratio in respect of m-amino-phenol of from 0.01 to 0.2.

12. A process as claimed in any of claims 1 to 11, characterized in that the organic phase comprises, in

addition to p-chloro-nitrobenzene, a solvent selected from aromatic and/or aliphatic hydrocarbons and chloroderivatives thereof.

13. A process as claimed in any of claims 1 to 12, characterized in that m-amino-phenol is in the form of a Na salt or of a K salt.

14. 3,4' -amino-nitro-diphenylether, when obtained according to the process claimed in any preceding claim.